**Europäisches Patentamt**

**European Patent Office**

**Office européen des brevets**

(19)

(11) Veröffentlichungsnummer: **0 147 645**
**B1**

(12) **EUROPÄISCHE PATENTSCHRIFT**

(45) Veröffentlichungstag der Patentschrift:
24.05.89

(21) Anmeldenummer: **84114296.1**

(22) Anmeldetag: **27.11.84**

(51) Int. Cl.⁴: **A 61 F 5/04**, A 61 F 5/02

(54) Einrichtung zur insbesondere postoperativen Behandlung eines Schulter- und/oder Ellbogengelenkes.

(30) Priorität: **15.12.83 DE 3345386**

(43) Veröffentlichungstag der Anmeldung:
**10.07.85 Patentblatt 85/28**

(45) Bekanntmachung des Hinweises auf die Patenterteilung:
**24.05.89 Patentblatt 89/21**

(84) Benannte Vertragsstaaten:
**AT BE CH DE FR GB IT LI NL SE**

(56) Entgegenhaltungen:
**US-A- 1 257 297**
**US-A- 1 639 815**
**US-A- 1 976 244**
**US-A- 4 280 490**
**US-A- 4 373 517**

(73) Patentinhaber: **Ernst Knoll GmbH, Feinmechanik,
Herrengässle 3, D-7801 Umkirch (DE)**

(72) Erfinder: **Blauth, Walter, Prof., Moltkestrasse 14,
D-2300 Kiel (DE)**
Erfinder: **Knoll, Ernst, Herrengässle 3, D-7801 Umkirch
(DE)**

(74) Vertreter: **Patentanwälte Dipl.-Ing. Hans Schmitt
Dipl.-Ing. Wolfgang Maucher, Dreikönigstrasse 13,
D-7800 Freiburg i.Br. (DE)**

## Beschreibung

Die Erfindung betrifft eine Einrichtung zur insbesondere postoperativen Behandlung eines Schulter- und/oder Ellbogengelenkes, mit einer den Arm unterstützenden Stützschiene, die über wenigstens ein Schulter-Schwenkgelenk an einem Trägergestell od.dgl. befestigt ist und ggf. ein Beugegelenk für Armbeugebewegungen im Ellbogenbereich aufweist, wobei das Trägergestell od.dgl. eine am Körper des Patienten befestigbare Halterung zumindest mit einer Befestigung im Schulterbereich aufweist.

Es sind bereits solche Einrichtungen bekannt, die z.B. unmittelbar an einem Stuhl für den Patienten oder an einem neben dem Patienten zu positionierenden Gestell angebracht sind. Nachteilig ist hier jedoch einerseits, daß der Patient bei der Behandlung verhältnismäßig starr an das ortsfeste Gerät gebunden ist. Andererseits ist durch die Trennung von Patient und Vorrichtung eine genau auf das Schultergelenk abgestimmte Bewegung zumindest problematisch, da bei Lageveränderungen des Patienten die Gelenkachsen der zu behandelnden Schulter und des Gerätes aus ihrer ausgerichteten, fluchtenden Stellung gelangen können. Außerdem ist der konstruktive Aufbau bei dieser Einrichtung vergleichsweise kompliziert.

Aus der US-A-1 257 297 kennt man bereits eine Armschiene, welche am Körper des Patienten befestigbar ist und über Gelenke miteinander verbundene, verstellbare Teile zum Unterstützen des Armes in unterschiedlichen Lagen aufweist.

Es handelt sich dabei um eine statische Schiene zum Ruhigstellen eines Armes in einer voreinstellbaren Position. Für Bewegungsübungen ist diese Armschiene nicht vorgesehen und wegen der Anordnung der Gelenke auch nicht geeignet. Es würden sich nämlich bei solchen Bewegungsübungen durch den Versatz zwischen Schulter- bzw. Ellbogengelenk und den entsprechenden Armschienengelenken unerwünschte Ausgleichsbewegungen in Ausheberichtung ergeben, welche das Schulter- bzw. Armgelenk erheblich belasten würden. Eine solche Zusatzbelastung ist jedoch höchst unerwünscht und könnte auch zu einer Verzögerung des Heilungsprozesses führen.

Auch die US-A-1 639 815 zeigt eine statische Armschiene zum Ruhigstellen eines Armes, welche aus den vorgenannten Gründen ebenfalls zum Durchführen von Bewegungsübungen nicht geeignet ist.

Aufgabe der vorliegenden Erfindung ist es, eine Einrichtung der eingangs erwähnten Art zu schaffen, bei welcher während der Durchführung von Bewegungsübungen unerwünschte Ausgleichsbewegungen und damit unerwünschte Zusatzbelastungen innerhalb des Schulter- bzw. Ellbogengelenkbereiches vermieden werden. Außerdem soll die einmal eingestellte Lage der Einrichtung mit ausgerichteten Gelenkachsen von Schulter- bzw. Ellbogengelenk und Bewegungs-Einrichtung erhalten bleiben.

Zur Lösung dieser Aufgabe wird vorgeschlagen, daß das oder die Schulter-Schwenkgelenke auf die Gelenkachsen der zu behandelnden Schulter einstellbar sind derat, daß ihre Gelenkachsen jeweils mit den entsprechenden Schultergelenkachsen des Patienten fluchten und daß wenigstens eines der Gelenke einen motorischen Bewegungsantrieb aufweist.

Durch die fluchtend einstellbare Lage der Schwenk-Gelenkachsen und des jeweiligen Gelenkes des Patienten werden Ausgleichsbewegungen im Schulter- oder Armgelenkbereich vermieden. Außerdem ist durch die Verstellbarkeit auch ein Ausrichten auf unterschiedlich große Patienten möglich.

Eine vorteilhafte Weiterbildung der Erfindung sieht vor, daß die Schulter-Befestigung zur Anbringung an der der zu behandelnden Schulter gegenüberliegenden, gesunden Schulter ausgebildet ist.

Die zu behandelnde Schulter bleibt somit während der Bewegungsübung von der Halterung der Einrichtung unbelastet. Somit ist der Einsatz der Einrichtung auch in Fällen möglich, wo eine Belastung des zu behandelnden Schulterbereiches durch eine Halterung noch nicht durchführbar ist.

Zweckmäßigerweise ist zwischen dem Trägergestell und der Stützschiene jeweils ein Gelenk mit Antrieb für eine etwa horizontale Schwenkbewegung sowie vorzugsweise ein weiteres Gelenk mit Antrieb für eine vertikale Auf- und Ab-Schwenkbewegung der Stützschiene vorgesehen. Dadurch können in vorteilhafter Weise Schwenkbewegungen in unterschiedlichen Bewegungsebenen durchgeführt werden.

Nach einer Weiterbildung der Erfindung sind das Schwenkgelenk für die etwa horizontale Schwenkbewegung oberhalb der Schulter und das für die etwa vertikale Schwenkbewegung rückenseitig oder brustseitig angeordnet. Dadurch ist einerseits die Zuordnung der Schwenkgelenkachsen auf die Position des Schultergelenkes möglich und außerdem ist der Patient durch diese Anordnung nur wenig behindert.

Zweckmäßigerweise weisen die Antriebe ein Gehäuse mit einem Befestigungsanschluß sowie einem Drehabtrieb auf und bilden selbst jeweils gleichzeitig ein Schwenkgelenk. Diese Antriebe können somit in vorteilhafter Weise gleich als Schwenkgelenk zwischen die relativ zueinander zu bewegenden Vorrichtungsteile eingebaut werden. Umständliche Übertragungsmechanismen werden dadurch vermieden.

Zweckmäßigerweise weist das Trägergestell od.dgl. eine am Körper des Patienten befestigbare Haltestütze auf, an der der Vertikal-Schwenkantrieb befestigt ist, wobei mit dessen Abtrieb der Horizontal-Schwenkantrieb verbunden ist.

Der Vertikal-Schwenkantrieb bewegt somit auch insgesamt den Horizontal-Schwenkantrieb, an dem die Stützschiene befestigt ist. Dadurch sind in beiden Schwenkebenen auch überlagerte Bewegungsabläufe durchführbar, die für krankengymnastische Übungen Vorteile bringen.

Gegebenenfalls kann ein Drehantrieb an der Stützschiene zum Verdrehen von deren Außenteil einschließlich des Beugegelenkes vorgesehen sein. Mit Hilfe dieses Antriebes ist dann zusätzlich zu den Schwenkbewegungen auch ein Verdrehen des Armes (Rotation) bzw. bei angebeugtem Arm ein Verschwenken des Unterarmes möglich.

Vorteilhafterweise sind die Schulter-Befestigung insbesondere zur Lagefixierung und eine weitere Befestigung im Hüft- und/oder Gesäßbereich als gewichtsentlastende Abstützung ausgebildet. Durch die Schulter-Befestigung bleiben die Gelenkachsen von Schulter und Gerät in einer ausgerichteten, fluchtenden Stellung und die untere Befestigung bildet im wesentlichen eine Abstützung zur Aufnahme des Eigengewichtes des Gerätes.

Eine Ausführungsform sieht vor, daß als untere Abstützung das untere Ende der Haltestütze mit einer Hüftabstützung, vorzugsweise einer Stützschale od.dgl. verbunden ist, die vorzugsweise durch einen Hüftgürtel gehalten ist.

Diese Ausführungsform erlaubt auch eine Laufbewegung bzw. eine Behandlung im Stehen des Patienten.

Nach einer anderen Ausführungsform der Erfindung ist vorgesehen, daß die untere Abstützung durch eine Sitzschale gebildet ist, die mit der Haltestütze vorzugsweise über ein biegeelastisches Zwischenteil verbunden ist. Diese Ausführungsform ist insbesondere für eine Behandlung im Sitzen des Patienten vorgesehen, wobei das biegeelastische Zwischenteil zwischen Haltestütze und Sitzschale einerseits Rumpfbewegungen des Patienten ermöglicht und wobei sich andererseits aber eine genügend gute, gewichtsentlastende Abstützung ergibt.

Zusätzliche Ausgestaltungen der Erfindung sind in den weiteren Unteransprüchen aufgeführt. Nachstehend ist die Erfindung mit ihren wesentlichen Einzelheiten anhand eines Ausführungsbeispieles in den Zeichnungen noch näher erläutert.

Es zeigt etwas schematisiert:

Fig. 1 eine Rückseitenansicht einer erfindungsgemäßen Einrichtung in Strecklage,

Fig. 2 eine Rückseitenansicht eines Drehantriebes im Ellbogengelenkbereich der Stützschiene,

Fig. 3 eine Aufsicht einer Stützschiene mit einem Teil des Trägergestelles,

Fig. 4 eine Teilseitenansicht einer erfindungsgemäßen Einrichtung gemäß der Schnittlinie IV–IV in Fig. 3,

Fig. 5 eine Teilrückansicht insbesondere eines Trägergestelles, das gegenüber der Darstellung in Fig. 1 zur Behandlung des linken Armes bzw. des linken Schultergelenkes angesetzt ist,

Fig. 6 eine Ansicht einer etwas abgewandelten Ausführungsform einer erfindungsgemäßen Einrichtung, die hier brustseitig angebracht ist,

Fig. 7 eine etwa Fig. 6 entsprechende Darstellung, hier jedoch mit etwas abgewandeltem Trägergestell,

Fig. 8 eine Teilansicht einer erfindungsgemäßen Einrichtung mit einem im Ellbogengelenk zur Beugung und Streckung angeordneten elektromotorischen Antrieb,

Fig. 9 eine etwa Fig. 8 entsprechende Darstellung, hier jedoch mit im Ellbogenbereich angebrachtem, elektromotorischen Antrieb für eine Rotationsbewegung im Schultergelenk und

Fig. 10 eine perspektivische Darstellung eines Antriebes.

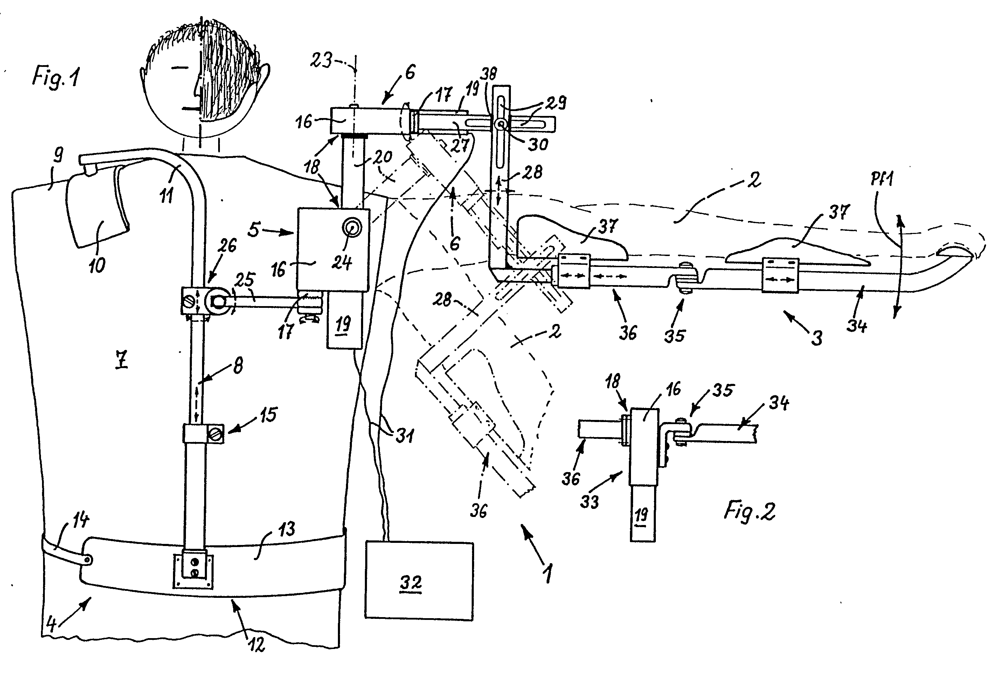

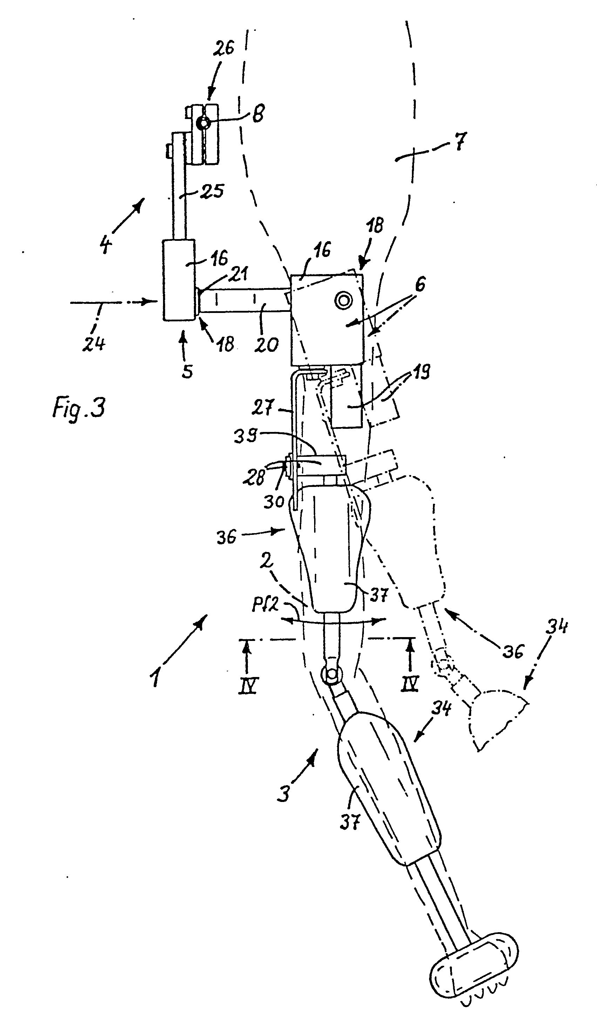

Eine Einrichtung 1 (Fig. 1, 3) dient zum Abstützen bzw. Unterstützen eines Armes 2, der in den Zeichnungen strichliniert umrissen ist. Ein solches Abstützen ist insbesondere nach Schultergelenk-Operationen erforderlich. Neben der Ruhigstellung des Armes 2 können mittels der Einrichtung 1 auch krankengymnastische Bewegungsübungen durchgeführt werden.

Die Einrichtung 1 weist im wesentlichen eine Stützschiene 3 sowie ein Trägergestell 4 auf. Dabei ist die Stützschiene 3 unter Zwischenschaltung von Schwenkantrieben 5 und 6 mit dem Trägergestell 4 verbunden. Mit Hilfe des Schwenkantriebes 5 kann eine vertikale Schwenkbewegung gemäß dem Doppelpfeil Pf 1 durchgeführt werden, während der Schwenkantrieb 6 für eine Horizontal-Schwenkbewegung gemäß dem Doppelpfeil Pf 2 in Fig. 3 dient. Strichpunktiert sind von der Strecklage abweichende Schwenklagen in den Fig. 1 und 3 eingezeichnet.

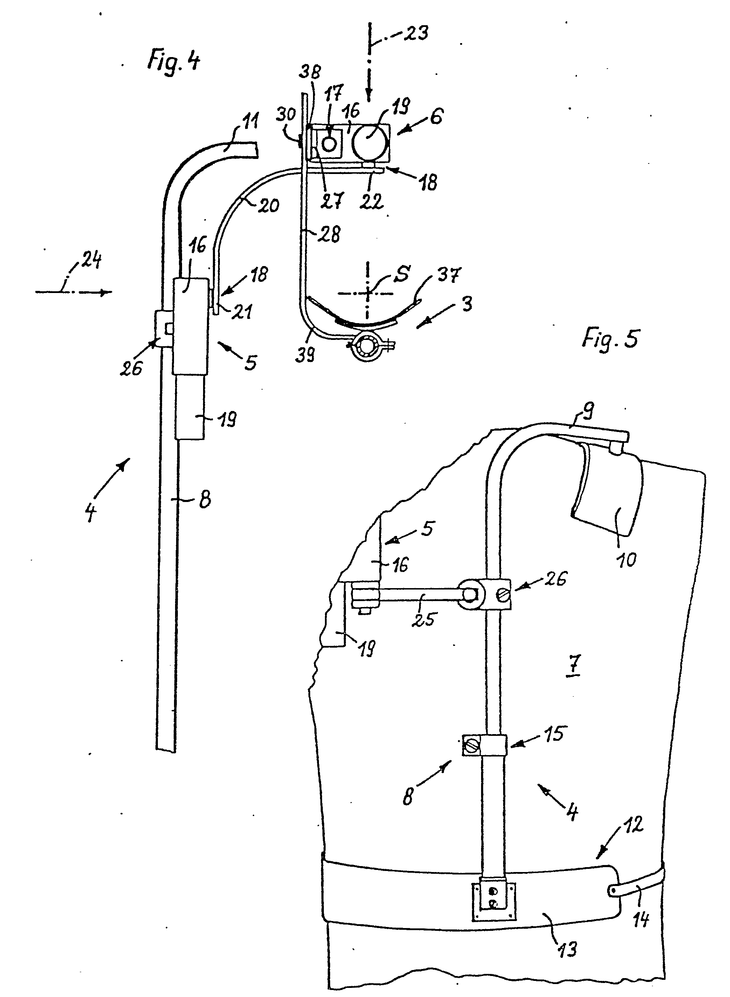

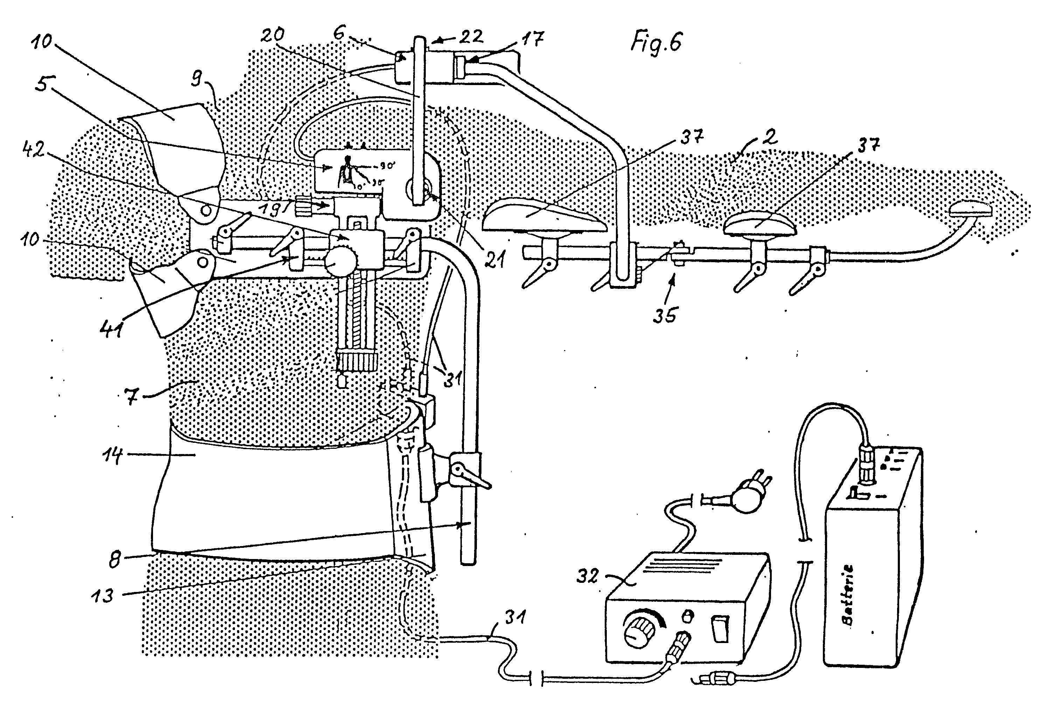

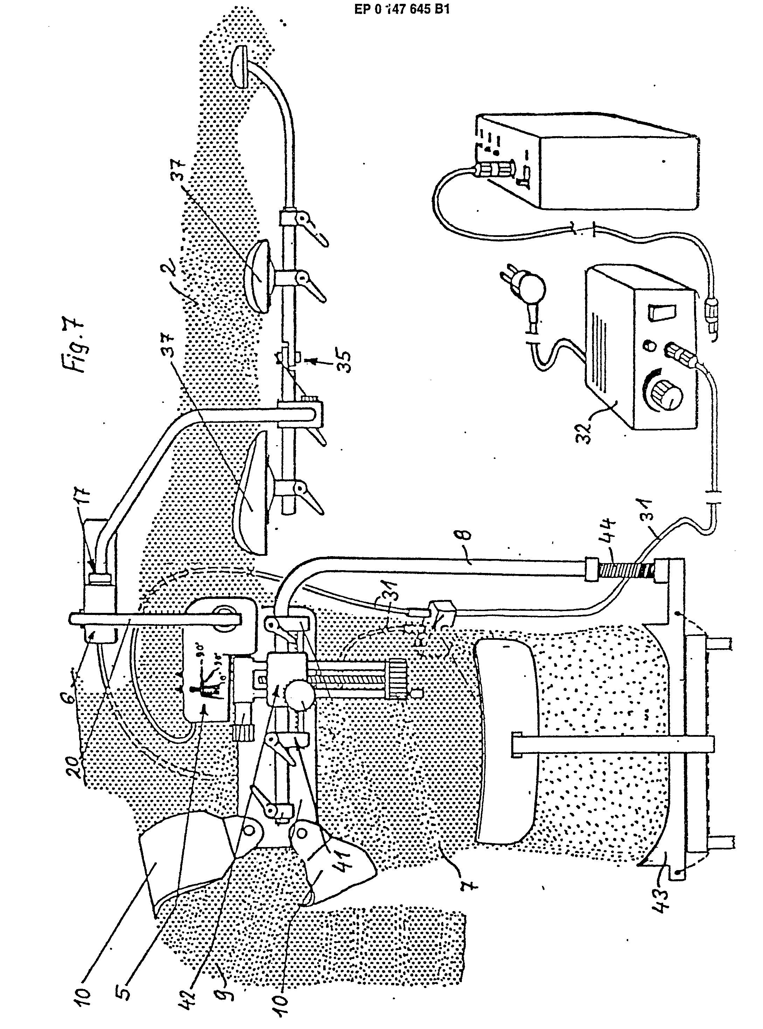

Das Trägergestell 4 weist eine am Körper des Patienten 7 befestigbare Haltestütze 8 auf, deren Enden mit Befestigungen im Hüftbereich sowie dem der Behandlungsseite abgewandten Schulterbereich verbunden sind. Das obere Ende der Haltestütze 8 ist abgewinkelt ausgebildet und ragt über die Halteschulter 9. Als Befestigung in diesem Schulterbereich ist eine diese Halteschulter 9 etwas umgreifende Stütze 10 vorgesehen, die mit der Abwinklung 11 der Haltestütze 8 vorzugsweise gelenkig verbunden ist. Gegebenenfalls kann hier zur Verbindung ein Kugelgelenk od.dgl. Schwenkgelenk vorgesehen sein. Zur Befestigung des unteren Endes der Haltestütze 8 ist ein Hüftgürtel 12 vorgesehen, der im wesentlichen aus einer etwa halbumfänglichen Stütz- und Haltemanschette 13 und einem sich daran anschließenden Riemen 14 besteht. Diese Ausbildung bzw. Anordnung des Hüftgürtels 12 ergibt einerseits eine stabile Abstützung der gesamten Einrichtung 1 und außerdem können dadurch die vom auftretenden Kippmoment herrührenden Kräfte gut auf den Körper, insbesondere Hüftbereich des Patienten übertragen werden. In Fig. 1 und 5 ist gut zu erkennen, daß die Stütz- und Haltemanschette 13 jeweils um die Hüfte gelegt ist, die sich auf der Schulter-Behandlungsseite befindet, da hier die vorerwähnten Andruckkräfte auftreten. Der Riemen 14 dient lediglich zum Zusammenhalten der Manschette 13. Die Haltestütze 8 ist zur Anpassung an unterschiedlich große Patienten teleskopartig in der Länge verstellbar und hier mittels einer Klemmvorrichtung 15 festlegbar ausgebildet. Gegebenenfalls könnte noch ein weiterer Haltegurt vorderseitig zwischen der Stütze 10 und der Manschette 13 bzw. um das gesunde Schultergelenk herum, wie in Fig. 6 und 7 gezeigt, vorgesehen sein.

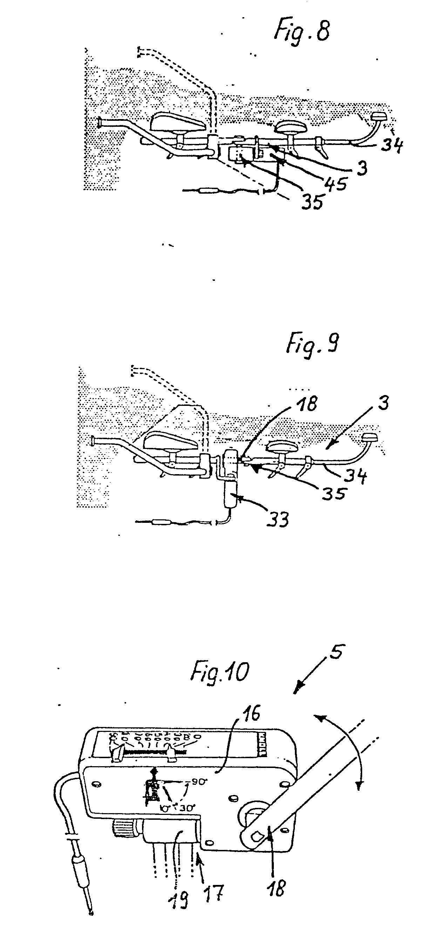

Die Schwenkantriebe 5 und 6 weisen jeweils ein Gehäuse 16 mit einem Befestigungsanschluß 17 sowie einem Drehabtrieb 18 auf (vgl. auch Fig. 4 und 10). In dem Gehäuse 16 befindet sich ein Getriebe, vorzugsweise ein Schneckengetriebe und außen sind Elektromotoren 19 an das Gehäuse 16 angeflanscht. Der Abtrieb 18 des Vertikal-Schwenkantriebes 5 ist über einen etwa die Schulter übergreifenden Winkelbügel 20 (vgl. Fig. 4), dessen Anschlußenden 21, 22 etwa rechtwinklig zueinander angeordnet sind, mit dem Abtrieb 18 des Horizontal-Schwenkantriebes 6 verbunden. Somit macht der Horizontal-Schwenkantrieb 6 die Vertikal-Schwenkbewegung der Stützschiene 3 bei Betätigung des Schwenkantriebes 5 mit. Durch diese Anordnung besteht auch die Möglichkeit, überlagerte Horizontal-Vertikal-Bewegungen durchzuführen, so daß Bewegungen des Armes über den gesamten, anatomisch vorgegebenen Bereich durchführbar sind. In Fig. 1 ist strichpunktiert eine Vertikal-Schwenkstellung eingezeichnet, bei der der Arm 2 etwas nach unten abgesenkt ist. In Fig. 3 zeigt die strichpunktierte Stellung eine etwas nach vorne geschwenkte Lage der Stützschiene 3.

Aus den Fig. 1, 3 und 4 ist gut zu erkennen, daß die Schwenkantriebe 5 und 6 so angeordnet sind, daß die Bewegungsachsen ihren Schnittpunkt S im Schultergelenk haben. Durch die strichpunktierten Pfeile 23 und 24 sind die Horizontal-Schwenkachse und die Vertikal-Schwenkachse, die jeweils durch die Drehabtriebe 18 verlaufen, angedeutet.

Der Vertikal-Schwenkantrieb 5 ist über eine Verbindungsstange 25 mit der Haltestütze 8 verbunden. Im Bereich der Haltestütze ist dabei ein Klemmstück 26 vorgesehen, das sich an der Haltestütze 8 höhenverstellen und festlegen läßt und wobei der Befestigungsanschluß für die Verbindungsstange 25 so ausgebildet ist, daß auch noch eine vertikale Verschwenkung und Festlegung der Stange 25 möglich ist. Außerdem läßt sich der Schwenkantrieb 5 bei seinem Befestigungsanschluß 17 gegenüber der Verbindungsstange 25 noch horizontal verdrehen und festlegen.

Durch die vorgenannten Maßnahmen ist ein genaues Ausrichten und Anpassen der Lage der Schwenkantriebe auf das Schultergelenk auch bei unterschiedlich großen Patienten möglich. An dem Befestigungsanschluß 17 des Schwenkantriebes 6 ist ein Bügel 27 befestigt, der mit einem weiteren, winklig dazu angeordneten Bügel 28 verbunden ist. Der erste Bügel 27 ist dabei etwa horizontal bei Strecklage des Armes orientiert, während der andere Bügel 28 etwa rechtwinklig dazu verläuft und an seinem unteren Ende eine Abwinklung 39 zum Untergreifen der Stützschiene 3 aufweist, wie dies gut in Fig. 4 erkennbar ist. Die beiden Bügel 27 und 28 sind in ihrem Verbindungsbereich relativ zueinander verstellbar. Dazu weisen die Bügel 27 und 28 Längsschlitze 29 auf, die übereinanderliegend von einer gemeinsamen Halteschraube 30 durchsetzt sind. Auch dadurch ist auf einfache Weise eine Lageanpassung der Stützschiene 3 an unterschiedliche Armlängen und Armdicken u. dgl. möglich. Im Verbindungsbereich der Bügel 27, 28 kann noch eine Kreuzführung 38 zum Halten der Bügel in winkliger Lage vorgesehen sein. In Fig. 1 ist angedeutet, daß die Motoren 18 über Anschlußkabel 31 mit einer Steuereinrichtung 32 verbunden sind. Diese Steuereinrichtung 32 kann einen Programmgeber, insbesondere für voreinstellbare Bewegungsabläufe, aufweisen. In vorteilhafter Weise kann dadurch die jeweils auf den Patienten abgestimmte Bewegungstherapie einprogrammiert werden und dann bedarfsweise, gegebenenfalls vom Patienten selbst abgerufen werden. Dadurch kann sich die Inanspruchnahme einer Fachkraft jeweils auf das Einstellen der Bewegungsabläufe beschränken, während dann die eigentliche Bewegungstherapie entsprechend dieser Voreinstellung automatisch ablaufen kann. Somit kann eine entsprechend geschulte Fachkraft (Krankengymnastin) wesentlich mehr Patienten in einer bestimmten Zeit behandeln. Gegebenenfalls ist dadurch auch eine Selbstbehandlung des Patienten zuhause möglich, so daß ein kostspieliger Krankenhausaufenthalt zumindest teilweise vermieden werden kann. Die Antriebe können jeweils gesteuerte Schrittmotoren aufweisen, mittels denen eine genügend genaue Positionierung der Stützschiene 3 möglich ist. Gegebenenfalls können aber auch bei den Antrieben Weggeber zur Positionsrückmeldung an die Steuereinrichtung 32 vorgesehen sein, so daß dadurch eine zusätzliche Kontroll-Rückmeldung und damit ein Regelkreis vorhanden ist. Zweckmäßig ist es dabei, wenn zur zusätzlichen Sicherheit gegen Überschreiten von vorgesehenen Bewegungsendstellungen bzw. auf den Schwenkbereich eines Armes etwa abgestimmte Schwenkbegrenzungen vorgesehen sind. Beispielsweise könnten dazu verstellbare Endanschläge, Endschalter, gegebenenfalls in Kombination mit Rutschkupplungen vorgesehen sein.

Wie bereits vorerwähnt, weisen die Schwenkantriebe 5 und 6 von den Motoren 19 angetriebene Getriebe auf, die insbesondere selbstsperrend, vorzugsweise als Schneckengetriebe ausgebildet sind. Dadurch bildet die Einrichtung 1 gleichzeitig bei abgeschalteten Antrieben eine herkömmliche Stützschiene zur Ruhigstellung des Armes, ohne daß dazu besondere Arretierungsmittel erforderlich sind. Außerdem wird durch die selbstsperrenden Getriebe verhindert, daß die Stützschiene 3 bei Stromausfall insbesondere nach unten unkontrolliert abschwenkt.

Fig. 2 und 9 zeigen noch eine Weiterbildung der Erfindung, mittels der auch eine Drehbewegung des Armes zusätzlich möglich ist. Dazu ist an der Stützschiene 3 ein Drehantrieb 33 angeordnet, durch den das Außenteil 34 der Stützschiene 3 einschließlich des Beugegelenkes 35 verdrehbar ist. Bei angebeugtem Unterarm würde dies ein Vertikalverschwenken von diesem bewirken. Der Drehantrieb 33 kann wie die Schwenkantriebe 5 und 6 mit einem Getriebe sowie einem Elektromotor 19 ausgebildet sein. Im Ausführungsbeispiel nach Fig. 2 ist der Drehabtrieb 18 mit dem Innenteil 36 der Stützschiene 3 verbunden, während am Gehäuse 16 das Beugegelenk 35 angeschlossen ist. In Fig. 9 ist der Drehabtrieb 18 mit dem Außenteil 34 bzw. dem Beugegelenk 35 verbunden.

Fig. 1 läßt gut erkennen, daß das Innenteil 36 der Stützschiene 3 teleskopartig längenverstellbar und festlegbar ausgebildet ist. Das Außenteil 34 und das Innenteil 36 der Stützschiene 3 tragen jeweils Armauflagen 37, die ebenfalls verstellbar angebracht sind.

Das untere Ende der Haltestütze 8 ist um 180° wendbar mit der Haltemanschette 13 verbunden, so daß durch diese Schwenkbewegung und durch ein Verdrehen des oberen Teiles der Haltestütze 8 wahlweise eine rechtsseitige bzw. linksseitige Anordnung des Trägergestelles 4 möglich ist. Erwähnt sei noch, daß anstatt des dargestellten Trägergestelles 4 auch ein korsettartiges Trägergestell oder ein aus dem Körper bereichsweise angepaßten Formhalbschalen od. dgl. bestehendes Trägergestell vorgesehen sein kann.

Das Trägergestell 4 kann, wie in Fig. 1 durch die halbseitige Kopfvorder- bzw. Rückseite angedeutet, sowohl am Rücken als auch auf der Brust des Patienten angebracht werden. Die Anbringung — vorne oder hinten — kann in Abhängigkeit von mehreren Gesichtspunkten erfolgen. Die Rückenmontage ist z.B. bei bestimmten erforderlichen Armbewegungen nach vorne vorteilhaft, weil dabei das am Rücken angebrachte Trägergestell 4 nicht hinderlich ist. Andererseits ist bei einer brustseitigen Montage vorteilhaft, daß sich der Patient ohne Behinderung setzen kann und daß auch gegebenenfalls erforderliche Anpaß-Vorstellbewegungen durch den Patienten selbst einfacher und unter Sichtkontrolle durchgeführt werden können. Eine solche Verstellbewegung könnte beispielsweise nach dem Setzen eines Patienten erforderlich sein, da hierbei eine Verkürzung des Schulter-Becken-Abstandes auftritt, die durch teleskopartiges Ineinanderschieben der Haltestütze 8 ausgeglichen und angepaßt werden kann. Außerdem ist diese Brust-Anbringung bei Patienten mit Rundrücken günstiger. Im Falle eines solchen Rundrückens könnte das Trägergestell 4 mit seiner Haltestütze 8 jedoch auch an die physiologische Krümmung des Rückens angepaßt sein und rückseitig angebracht werden. Eine solche Formanpassung ist auch an die Form des Brustkorbes bei brustseitiger Anbringung möglich.

Erwähnt sei noch, daß neben der, wie in Fig. 1 gezeigten mittigen Anordnung der Haltestütze 8, diese auch erforderlichenfalls seitenversetzt unsymmetrisch angeordnet werden kann. Eine solche Ausführungsform zeigen die Fig. 6 und 7. Auch bei dieser Anordnung ist eine Fixierung an der gesunden Halteschulter 9 sowie eine Abstützung im Hüft- oder Gesäßbereich vorgesehen. Durch diese Befestigung direkt am Patienten wird weitgehend vermieden, daß Bewegungen des Patienten zu einer Verstellung der ausgerichteten Lage der Gelenkachsen von Schulter und Gerät führen.

Die gegenüber Fig. 1 etwas abgewandelte Ausführungsform nach Fig. 6 weist ebenfalls eine hüftseitige Abstützung mit einer Stützmanschette 13 sowie einem Haltegurt 14 auf. Die schulterseitige Fixierung erfolgt hier mit einem die gesunde Halteschulter 9 umgreifenden Schultergurt 40. Weiterhin erkennt man bei dieser Ausführungsform zur Feineinstellung des Armlagerungsgestells in horizontaler und vertikaler Richtung eine Schiebehalterung 41 sowie ein Verstellgetriebe 42, mittels dem eine Vertikalhub-Einstellbewegung durchgeführt werden kann. Das Verstellgetriebe 42 ist zweckmäßigerweise als Spindelgetriebe ausgebildet, weil damit leichtgängig die gesamte Stützschiene 3 bewegt werden kann.

Die Ausführungsform nach Fig. 7 unterscheidet sich von der in Fig. 6 dargestellten dadurch, daß die untere Abstützung eine Sitzschale 43 aufweist, an der die Haltestütze 8 über ein biegeelastisches Zwischenteil 44 angeschlossen ist. Diese Ausführungsform ermöglicht eine Behandlung eines Patienten im Sitzen. Auch durch diese Ausbildung erfolgt eine gewichtsentlastende, Rumpfbewegungen zulassende Abstützung. Durch die Schulterhalterung mit dem Schultergut bleibt jedoch die Fixierung am Körper erhalten.

Fig. 8 zeigt noch eine Stützschiene 3, bei der im Ellbogenbereich bei dem Beugegelenk 35 ein weiterer Schwenkantrieb 45 zur Beugung und Streckung des Unterarmes angeordnet ist. Somit kann die erfindungsgemäße Einrichtung auch gegebenenfalls zusätzlich zur Behandlung des Ellbogengelenkes eingesetzt werden.

Die Anbringung der Schulterfixierung an der gesunden Halteschulter hat den erheblichen Vorteil, daß die zu trainierende Schulter nicht belastet wird, so daß gegebenenfalls wesentlich früher insbesondere nach Operationen mit einer Bewegungstherapie begonnen werden kann. Unter besonderen Umständen kann die Schulterfixierung 10 jedoch auch auf der zu behandelnden Schulter aufliegen.

**Patentansprüche**

1. Einrichtung (1) zur insbesondere postoperativen Behandlung eines Schulter- und/oder Ellbogengelenkes, mit einer den Arm (2) unterstützenden Stützschiene (3), die über wenigstens ein Schulter-Schwenkgelenk an einem Trägergestell (4) od. dgl. befestigt ist und ggf. ein Beugegelenk (35) für Armbeugebewegungen im Ellbogenbereich aufweist, wobei das Trägergestell (4) od. dgl. eine am Körper des Patienten (7) befestigbare Halterung zumindest mit einer Befestigung (10) im Schulterbereich aufweist, dadurch gekennzeichnet, daß das oder die Schulter-Schwenkgelenke auf die Gelenkachsen der zu behandelnden Schulter einstellbar sind derart, daß ihre Gelenkachsen (24, 23) jeweils mit den entsprechenden Schultergelenkachsen des Patienten (7) fluchten und daß wenigstens eines der Gelenke einen motorischen Bewegungsantrieb aufweist.

2. Einrichtung nach Anspruch 1, dadurch gekennzeichnet, daß die Schulter-Befestigung (10) zur Anbringung an der der zu behandelnden Schulter gegenüberliegenden, gesunden Schulter (9) ausgebildet ist.

3. Einrichtung nach einem der Ansprüche 1 oder 2, dadurch gekennzeichnet, daß zwischen dem Trägergestell (4) und der Stützschiene (3) jeweils ein Gelenk mit Antrieb für eine etwa horizontale Schwenkbewegung sowie vorzugsweise ein weiteres Gelenk mit Antrieb für eine vertikale Auf- und Ab-Schwenkbewegung der Stützschiene (3) vorgesehen ist.

4. Einrichtung nach einem der Ansprüche 1 bis 3, dadurch gekennzeichnet, daß das Trägergestell (4) od.dgl. eine am Körper des Patienten befestigbare Haltestütze (8) aufweist, an der der Vertikal-Schwenkantrieb (5) befestigt ist, und daß mit dessen Abtrieb (18) der Horizontal-Schwenkantrieb (6) verbunden ist.

5. Einrichtung nach einem der Ansprüche 1 bis 4, dadurch gekennzeichnet, daß die Schulter-Befestigung (10) insbesondere zur Lagefixierung und eine weitere Befestigung (13, 14) im Hüft- und/oder Gesäßbereich als gewichtsentlastende Abstützung ausgebildet sind.

6. Einrichtung nach einem der Ansprüche 1 bis 5, dadurch gekennzeichnet, daß das Schwenkgelenk für die etwa horizontale Schwenkbewegung oberhalb der Schulter und das für die etwa vertikale Schwenkbewegung rückenseitig oder brustseitig angeordnet sind.

7. Einrichtung nach einem der Ansprüche 1 bis 6, dadurch gekennzeichnet, daß die Antriebe für die Schwenkgelenke jeweils ein selbstsperrendes Getriebe, vorzugsweise ein Schneckengetriebe aufweisen, das zweckmäßigerweise mit einem Elektromotor (19) verbunden ist.

8. Einrichtung nach einem der Ansprüche 1 bis 7, dadurch gekennzeichnet, daß die Antriebe (5, 6, 33) mit einer Steuereinrichtung (32) verbunden sind, die vorzugsweie einen Programmgeber für voreinstellbare Bewegungsabläufe aufweist und daß die Antriebe (5, 6, 33) gegebenenfalls jeweils einen Weggeber zur Positionsrückmeldung an die Steuereinrichtung (32) aufweisen und daß gegebenenfalls die Schwenkantriebe (5, 6, 33) auf den Schwenkbereich bzw. Drehbereich eines Armes etwa abgestimmte Schwenkbegrenzungen, z.B. Endanschläge und/oder gegebenenfalls Endschalter od.dgl., bzw. Rutschkupplungen aufweisen.

9. Einrichtung nach einem der Ansprüche 1 bis 6, dadurch gekennzeichnet, daß die Antriebe (5, 6, 33) ein Gehäuse (16) mit einem Befestigungsanschluß (17) sowie einem Drehabtrieb (18) aufweisen und selbst jeweils gleichzeitig das Schwenkgelenk bilden.

10. Einrichtung nach einem der Ansprüche 1 bis 9, dadurch gekennzeichnet, daß der Abtrieb (18) des Vertikal-Schwenkantriebes (5) über einen etwa die Schulter übergreifenden Winkelbügel (20), dessen Anschlußenden (21, 22) etwa rechtwinklig zueinander angeordnet sind, mit dem Abtrieb (18) des Horizontal-Schwenktriebes (6) verbunden ist.

11. Einrichtung nach einem der Ansprüche 1 bis 10, dadurch gekennzeichnet, daß an dem Befestigungsanschluß (17) des Horizontal-Schwenkantriebes (6) wenigstens ein Verbindungsbügel (27, 28) od.dgl. zum Halten der Stützschiene (3) befestigt ist.

12. Einrichtung nach einem der Ansprüche 1 bis 11, dadurch gekennzeichnet, daß die Befestigungsanschlüsse (17) der Antriebe (5, 6) in Drehrichtung quer zur Abtriebsachse verstellbare Halter aufweisen.

13. Einrichtung nach einem der Ansprüche 1 bis 12, dadurch gekennzeichnet, daß ein Drehantrieb (33) an der Stützschiene (3) zum Verdrehen von deren Außenteil (34) einschließlich des Beugegelenkes (35) vorgesehen ist.

14. Einrichtung nach einem der Ansprüche 1 bis 13, dadurch gekennzeichnet, daß die Haltestütze (8) als Stange mit einer oberen, in Funktionsstellung eine Schulter etwa übergreifenden Abwinkelung (11) ausgebildet ist, deren freies, oberes Ende mit einer die Schulter (9) vorzugsweise zumindest etwas umgreifenden Stütze (10) verbunden ist.

15. Einrichtung nach einem der Ansprüche 1 bis 14, dadurch gekennzeichnet, daß die Befestigung zwischen Haltestütze (8) und Stütz- und Haltemanschette (13) um etwa 180° wendbar ist.

16. Einrichtung nach einem der Ansprüche 1 bis 15, dadurch gekennzeichnet, daß die Haltestütze (8) teleskopartig in der Länge verstellbar und festlegbar ausgebildet ist.

17. Einrichtung nach einem der Ansprüche 1 bis 16, dadurch gekennzeichnet, daß zwischen der Haltestütze (8) und dem Vertikal-Schwenkantrieb (5) ein Verbindungsstück vorgesehen ist, das an der Haltestütze (8) höhenverstellbar sowie vertikal schwenkbar und an dem Schwenkantrieb horizontal schwenkbar befestigt ist.

18. Einrichtung nach einem der Ansprüche 1 bis 17, dadurch gekennzeichnet, daß das Trägergestell (4) korsettartig ausgebildet ist oder aus dem Körper bereichsweise angepaßten Formhalbschalen besteht.

19. Einrichtung nach einem der Ansprüche 1 bis 18, dadurch gekennzeichnet, daß das Trägergestell (4) wahlweise rückenseitig oder brustseitig, gegebenenfalls außermittig am Patienten anordenbar ist.

20. Einrichtung nach einem der Ansprüche 1 bis 19, dadurch gekennzeichnet, daß das Trägergestell (4), insbesondere mit seiner Haltestütze (8), in Anpassung an die physiologische Krümmung des Rückens bzw. des Brustkorbes eines zu behandelnden Patienten gekrümmt ausgebildet ist.

21. Einrichtung nach einem der Ansprüche 1 bis 20, dadurch gekennzeichnet, daß als untere Abstützung das untere Ende der Haltestütze (8) mit einer Hüftabstützung, vorzugsweise einer Stützschale (13) od.dgl. verbunden ist, die vorzugsweise durch einen Hüftgürtel (14) gehalten ist.

22. Einrichtung nach einem der Ansprüche 1 bis 20, dadurch gekennzeichnet, daß die untere Abstützung durch eine Sitzschale (43) gebildet ist, die mit der Haltestütze (8) vorzugsweise über ein biegeelastisches Zwischenteil (44) verbunden ist.

23. Einrichtung nach einem der Ansprüche 1 bis 22, dadurch gekennzeichnet, daß zum Lageeinjustieren der Gelenkachsen zumindest zwischen der Haltestütze (8) und dem Antrieb für die vertikale Auf- und Ab-Schwenkbewegung, ein Verstellgetriebe (42), vorzugsweise ein Spindelgetriebe vorgesehen ist.

**Claims**

1. A device (1), especially for the postoperative treatment of a shoulder and/or elbow joint, including a supporting splint (3) which props up the arm (2), is fastened to a carrying frame (4) or the like by way of a least one shoulder swivel joint and may have a joint (35) for bending movements of the arm in the elbow region, the carrying frame (4) or the like having a holder which is adapted to be fastened to the body of the patient (7) and includes at least one fixation (10) in the shoulder region, characterized in that the shoulder swivel joint(s) are adjustable to the joint axes of the shoulder under treatment in such a manner that the joint axes (24, 23) of the former are in each case in alignment with the corresponding shoulder joint axes of the patient (7) and that at least one of the joints has a motor-operated drive for movement.

2. The device as claimed in claim 1, characterized in that the shoulder fixation (10) is devised to be applied to the healthy shoulder (9) opposite the one under treatment.

3. The device as claimed in either of claims 1 or 2, characterized in that between the carrying frame (4) and the supporting splint (3) provision is made in each case for a joint with a drive for an approximately horizontal swivel movement and preferably a further joint with a drive for a vertical, up and down swivel movement of the supporting splint (3).

4. The device as claimed in any one of claims 1 to 3, characterized in that the carrying frame (4) or the like has a stay (8) which is adapted to be fastened to the patient's body and has the vertical-swivel drive (5) fixed to it, and that the horizontal-swivel drive (6) is connected to the output (18) of the latter.

5. The device as claimed in any one of claims 1 to 4, characterized in that the shoulder fixation (10) is devised particularly for setting the position and a further fixation (13, 14) in the region of the hip and/or posterior is devised to be a weight-relieving support.

6. The device as claimed in any one of claims 1 to 5, characterized in that the swivel joint for the approximately horizontal swivel movement is disposed above the shoulder and the swivel joint for the approximately vertical swivel movement is disposed at the back or at the chest.

7. The device as claimed in any one of claims 1 to 6, characterized in that the drives for the swivel joints in each case have a self-locking gear, preferably a worm gear, which suitably is connected to an electromotor (19).

8. The device as claimed in any one of claims 1 to 7, characterized in that the drives (5, 6, 33) are connected to a control device (32) preferably having a programme transmitter for presettable sequences of movement and that the drives (5, 6, 33) may in each case have a position indicator for positional feedback to the control device (32) and that the swivel drives (5, 6, 33) may have limiting means co-ordinated approximately to the range in which an arm swivels or turns, e.g. limit stops and/or possibly limit switches of the like, and friction couplings.

9. The device as claimed in any one of claims 1 to 6, characterized in that the drives (5, 6, 33) have a housing (16) with a mounting attachment (17) as well as a rotary output (18) and in each case at the same time themselves from the swivel joint.

10. The device as claimed in any one of claims 1 to 9, characterized in that the output (18) of the vertical-swivel drive (5) is connected to the output (18) of the horizontal-swivel drive (6) by way of an angled bar (20) engaging over the shoulder and having its connecting ends (21, 22) disposed approximately at right angles to one another.

11. The device as claimed in any one of claims 1 to 10, characterized in that at least one bow (27, 28) or the like is fastened to the mounting attachment (17) of the horizontal-swivel drive (6) and serves to hold the supporting splint (3).

12. The device as claimed in any one of claims 1 to 11, characterized in that the mounting attachments (17) of the drives (5, 6) have holders adjustable transversely to the output axis in the direction of rotation.

13. The device as claimed in any one of claims 1 to 12, characterized in that a rotary drive (33) is provided on the supporting splint (3) to twist the outer part (34) of the latter including the joint (35) for bending.

14. The device as claimed in any one of claims 1 to 13, characterized in that the stay (8) takes the form of a rod with an upper bend (11) which, in the position for use, engages over a shoulder, the free, upper end thereof being connected to a support (10) which preferably enclasps the shoulder (9) at least to some extent.

15. The device as claimed in any one of claims 1 to 14, characterized in that the fixation between stay (8) and supporting and holding collar (13) is reversible through approximately 180°.

16. The device as claimed in any one of claims 1 to 15, characterized in that the stay (8) is adapted to be telescopically adjusted in length and fixed.

17. The device as claimed in any one of claims 1 to 16, characterized in that a connecting piece is provided between the stay (8) and the vertical-swivel drive (5), said connecting piece being fastened to the stay (8) so as to be vertically adjustable and capable of swivelling vertically and being fastened to the swivel drive so as to be capable of swivelling horizontally.

18. The device as claimed in any one of claims 1 to 17, characterized in that the carrying frame (4) is devised to be corset-like or consists of moulded half- shells adapted to the body in areas.

19. The device as claimed in any one of claims 1 to 18, characterized in that the carrying frame (4) is adapted to be disposed at the back or at the chest of the patient selectively, possibly off-centre.

20. The device as claimed in any one of claims 1 to 19, characterized in that the carrying frame (4), particularly with the stay (8) thereof is devised to be curved in adaptation to the physiological curvature of the back or rib cage of the patient under treatment.

21. The device as claimed in any one of claims 1 to 20, characterized in that in the form of a lower support the lower end of the stay (8) is connected to a hip support, preferably a supporting pan (13) or the like preferably held by a girdle (14).

22. The device as claimed in any one of claims 1 to 20, characterized in that the lower support is formed by a dished seat (43) connected to the stay (8) preferably by way of a flexible intermediate part (44).

23. The device as claimed in any one of claims 1 to 22, an adjusting gear (42), preferably a spindle gear, is provided for positionally adjusting the joint axes at least between the stay (8) and the drive for the vertical, up and down swivel movement.

**Revendications**

1. Dispositif (1) notamment destiné au traitement postopératoire d'une articulation scapulaire et/ou cubitale, comportant une atelle (3) qui soutient le bras (2), est fixée à un châssis de support (4) ou structure similaire par l'intermédiaire d'au moins une articulation pivotant sur l'épaule, et possède éventuellement une articulation de flexion (35) pour des mouvements de fléchissement du bras dans la région cubitale, le châssis de support (4) ou structure similaire présentant un système de retenue pouvant être assujetti au corps du patient (7), avec au moins une fixation (10) dans la région scapulaire, caractérisé par le fait que l'articulation ou les articulations pivotant sur l'épaule peuvent être réglées en fonction des axes d'articulation de l'épaule devant être traitée, de manière que leurs axes d'articulation (24, 23) coïncident respectivement avec les axes correspondants d'articulation scapulaire du patient (7); et par le fait qu'au moins l'une des articulations présente un entraînement motorisé imprimant des mouvements.

2. Dispositif selon la revendication 1, caractérisé par le fait que la fixation (10) à l'épaule est réalisée en vue de l'installation sur l'épaule saine (9) opposée à l'épaule devant être traitée.

3. Dispositif selon l'une des revendications 1 ou 2, caractérisé par le fait qu'il est respectivement prévu, entre le châssis de support (4) et l'attelle (3), une articulation à entraînement pour un mouvement pivotant sensiblement horizontal, ainsi que, de préférence, une autre articulation à entraînement pour un pivotement alternativ vertical de l'attelle (3).

4. Dispositif selon l'une des revendications 1 à 3, caractérisé par le fait que le châssis de support (4) ou structure similaire présente un étai de retenue (8) pouvant être fixé au corps du patient, et auquel l'entraînement (5) en pivotements verticaux est fixé; et par le fait que l'entraînement (6) en pivotements horizontaux est relié à la prise de sortie (18) de l'entraînement précité.

5. Dispositif selon l'une des revendications 1 à 4, caractérisé par le fait que la fixation (10) à l'épaule est notamment réalisée en vue de la consignation à demeure, une fixation supplémentaire (13, 14) dans la région coxale et/ou fessière étant réalisée en tant que soutien de soulagement pondéral.

6. Dispositif selon l'une des revendications 1 à 5, caractérisé par le fait que l'articulation pivotante destinée au pivotement sensiblement horizontal est implantée au-dessus de l'épaule, et celle destinée au pivotement sensiblement vertical se trouve côté dorsal au côté pectoral.

7. Dispositif selon l'une des revendications 1 à 6, caractérisé par le fait que les entraînements des articulations pivotantes présentent respectivement un train autobloquant, de préférence en engrenage à vis sans fin qui est commodément relié à un moteur électrique (19).

8. Dispositif selon l'une des revendications 1 à 7, caractérisé par le fait que les entraînements (5, 6, 33) sont reliés à un dispositif de commande (32) préférentiellement muni d'un programmateur pour des processus moteurs préréglables; par le fait que les entraînements (5, 6, 33) comportent respectivement, le cas échéant, un indicateur de courses assurant la signalisation rétroactive de positions au dispositif de commande (32); et par le fait que les entraînements en pivotement (5, 6, 33) comportent éventuellement des organes limiteurs de pivotements, par exemple des butées extrêmes et/ou éventuellement des interrupteurs de fin de course ou organes similaires, respectivement des accouplements patinants, sensiblement harmonisés avec la plage respective de pivotements ou de rotations d'un bras.

9. Dispositif selon l'une des revendications 1 à 6, caractérisé par le fait que les entraînements (5, 6, 33) présentent un carter (16) comprenant un raccord de fixation (17) ainsi qu'un entraînement en rotation (18), et forment eux-mêmes simultanément, à chaque fois, l'articulation pivotante.

10. Dispositif selon l'une des revendications 1 à 9, caractérisé par le fait que la prise de sortie (18) de l'entraînement (5) en pivotements verticaux est reliée à la prise de sortie (18) de l'entraînement (6) en pivotements horizontaux, par l'intermédiaire d'un étrier en cornière (20) qui emprisonne substantiellement l'épaule par-dessus, et dont les extrémités de solidarisation (21, 22) sont disposées sensiblement à angle droit l'une par rapport à l'autre.

11. Dispositif selon l'une des revendications 1 à 10, caractérisé par le fait qu'au moins un étrier de liaison (27, 28) ou élément similaire, destiné à retenir l'attelle (3), est fixé au raccord de fixation (17) de l'entraînement (6) en pivotements horizontaux.

12. Dispositif selon l'une des revendications 1 à 11, caractérisé par le fait que les raccords de fixation (17) des entraînements (5, 6) présentent des supports réglables, dans le sens de rotation, transversalement par rapport à l'axe d'entraînement de sortie.

13. Dispositif selon l'une des revendications 1 à 12, caractérisé par le fait qu'un entraînement en rotation (33) est prévu sur l'attelle (3), pour faire tourner la partie extérieure (34) de celle-ci y compris l'articulation de flexion (35).

14. Dispositif selon l'une des revendications 1 à 12, caractérisé par le fait que l'étai de retenue (8) est réalisé sous la forme d'une tige présentant un cintrage supérieur (11) qui chevauche sensiblement une épaule dans la position de fonctionnement, et dont l'extrémité supérieure libre est reliée à un appui (10) qui, de préférence, ceinture au moins sensiblement l'épaule (9).

15. Dispositif selon l'une des revendications 1 à 14, caractérisé par le fait que la fixation entre l'étai de retenue (8) et le harnais (13) de soutien et de retenue peut tourner d'environ 180°.

16. Dispositif selon l'une des revendications 1 à 15, caractérisé par le fait que l'étai de retenue (8) est réalisé de longueur réglable et arrêtable télescopiquement.

17. Dispositif selon l'une des revendications 1 à 16, caractérisé par le fait qu'une pièce de liaison, prévue entre l'étai de retenue (8) et l'entraînement (5) en pivotements verticaux, est fixée à l'étai de retenue (8) avec faculté de réglage en hauteur et de pivotement vertical, et à l'entraînement en pivotements avec faculté de pivotement horizontal.

18. Dispositif selon l'une des revendications 1 à 17, caractérisé par le fait que le châssis de support (4) est réalisé à la manière d'un corset, ou bien consiste en des demi-coquilles moulées adaptées au corps par zones.

19. Dispositif selon l'une des revendications 1 à 18, caractérisé par le fait que le châssis de support (4) peut être sélectivement implanté côté dorsal au côté pectoral, éventuellement de manière excentrée sur le patient.

20. Dispositif selon l'une des revendications 1 à 19, caractérisé par le fait que le châssis de support (4) est réalisé, notamment par son étai de retenue (8), avec une courbure adaptée à la courbure physiologique du dos ou de la cage thoracique d'un patient devant être traité.

21. Dispositif selon l'une des revendications 1 à 20, caractérisé par le fait que l'extrémité inférieure de l'étai de retenue (8), faisant fonction de soutien inférieur, est reliée à un soutient iliaque, de préférence à une coquille de soutien (13) ou élément similaire préférentiellement retenu(e) par l'intermédiaire d'une sangle iliaque (14).

22. Dispositif selon l'une des revendications 1 à 20, caractérisé par le fait que le soutien inférieur est formé par une coquille fessière (43) reliée, à l'étai de retenue (8), de préférence au moyen d'une pièce intercalaire (44) ployant élastiquement.

23. Dispositif selon l'une des revendications 1 à 22, caractérisé par le fait qu'un train de réglage (42), de préférence un train à broche, est prévu pour le réglage des positions des axes d'articulation, au moins entre l'étai de retenue (8) et l'entraînement provoquant le pivotement alternatif vertical.

Fig.1

Fig.2

EP 0 147 645 B1

Fig.3

Fig. 4

Fig. 5

Fig.6

Fig. 7

Fig. 8

Fig. 9

Fig. 10